# EUROPEAN PATENT APPLICATION

(11) **EP 1 782 729 A1**
(43) Date of publication of application: **09.05.2007**
(21) Application number: 05425772.0
(22) Date of filing: 02.11.2005
(51) Int. Cl.: A61B 5/0245

(54) **Heart rate measurement and storage device, for sports activities, physical exercise and rehabilitation**

(71) Applicant: Hosand Technologies S.r.l., 28924 Verbania (IT)
(72) Inventor: Lopez Torres, Fernando, 08292 Esparraguera-Barcelona (ES)
(74) Representative: Petruzziello, Aldo

(57) **Abstract**

A device (100) for measuring and storing a user's heart rate for sports activities, physical exercise and rehabilitation comprises:
- a rigid case (1) containing a printed circuit card (5) on which are mounted a microcontroller (5), a memory (56), an IRDA interface (55), three LEDs (46, 47, 48), two switches (14, 15) and the contacts for the battery (6);
- a tentacle (2) of semi-rigid flexible material, connected to said rigid case (1);
- two electrocardiogram electrodes (3) having an adhesive bottom surface (32) to adhere to the user's skin and connection means (30) disposed on the top surface to be connected removeably with complementary connection means (12, 22) disposed respectively in the rigid case (1) and at the end of the tentacle (2). The electrodes (3) have an electrically conductive area (33) connected electrically to the microcontroller (5) to measure the electrocardiographic signal (ECG) by means of the difference in potential that is generated between the two electrodes (3) and store the derived heart beat value together with the temperature value in the memory (56).

## Description

The present invention refers to a device for measuring and storing heart rate, for sports activities, physical exercise and rehabilitation.

Measurement and storage of heart rate is the basic tool for obtaining reliable information on the internal work done, metabolic zones of work, stress level and other significant indicators in order to plan training programmes and monitor progress thereof.

Heart rate is measured through the person's skin, on the basis of an electrocardiographic signal generated by the heart beat. The electrical signal in turn is detected through electrodes (at least two) which adhere to the skin.

In the state of the art, the electrodes are positioned inside a chest strap which adheres to the individual's chest. An elastic strip attached to the ends of the band allows the strap to be tightened around the torso.

An electronic transmitter circuit connected to the electrodes is mounted in a central position within the strap. The electronic circuit intercepts the electrical signal generated by the heart beat and detected by the electrodes and transmits it, via radio, to an external receiving device (typically a receiver mounted inside a wrist watch or inside a fitness machine). The instant heart rate value, calculated by processing the time elapsed between successive events (successive beats) can be visualized on the display of the watch or of another device.

Sometimes the strap itself can include the ability to store heart rate data. Furthermore some models can be interfaced with an external device in order to download heart rate data to a device able to process them, typically a PC.

The chest strap is made of plastic or rubber. It is rigid at the front and elastic at the back. The chest strap is not suitable for prolonged use and can easily irritate the subject's skin.

Some people cannot easily wear the strap for various reasons (large muscle mass, large breasts, excessive thinness, etc.), since they encounter difficulty in finding the optimal position to obtain a good electrical signal.

Therefore the chest strap presents two serious disadvantages: it is difficult to position the rigid part of the strap around the torso in order to pick up the electrical signal and it can easily irritate the skin.

Moreover a further drawback presented by current devices is based on the fact that the man-machine interface for displaying, processing and sometimes transferring to a more powerful computing device is represented by a watch worn on the wrist. Functions such as marking of an event (for example the end of a lap), acoustic warnings in the event of exceeding the programmed upper or lower heart-rate thresholds, can be done only through an additional device such as the watch.

In addition, most commercially available chest straps do not have a data storage capability and even if some models perform this function, the heart rate measurement and storage modalities cannot be configured.

The object of the invention is to provide a heart rate measurement device that solves the above mentioned problems of the prior art.

Another object of the present invention is to provide such a heart rate measurement device that is not bulky and is comfortable for the user.

These objects are achieved in accordance with the invention with the characteristics listed in appended independent claim 1.

Advantageous embodiments of the invention are apparent from the dependent claims.

According to the invention the device for measuring and storing a user's heart rate for sports activities, physical exercise and rehabilitation, comprises:
- a rigid case containing a printed circuit card on which a microcontroller and a memory are mounted;
- a tentacle of semi-rigid flexible material, connected to the rigid case;
- two electrocardiogram electrodes having an adhesive bottom surface to adhere to the user's skin and connection means disposed on the top surface to connect removeably, with complementary connecting means disposed in the rigid case and at the end of the tentacle, respectively.

The electrodes have an electrically conductive area connected electrically to the microcontroller to measure the electrocardiographic (ECG) signal by means of the difference in potential that is generated between the two electrodes and store it in said memory.

The advantages of the device according to the invention, which uses traditional disposable electrodes that are connected as needed to the main body of the device, are evident. Furthermore, the provision of a microcontroller and a memory allows operation of the device according to the invention even without the use of a receiving device such as a wrist watch. In fact the electrocardiographic signal detected by the electrodes is stored in the memory from which it can be downloaded to any device able to process and display it.

Further characteristics of the invention will be made clear by the detailed description that follows, referring to a purely exemplifying and therefore non-limiting embodiment thereof, illustrated in the appended drawing, in which:

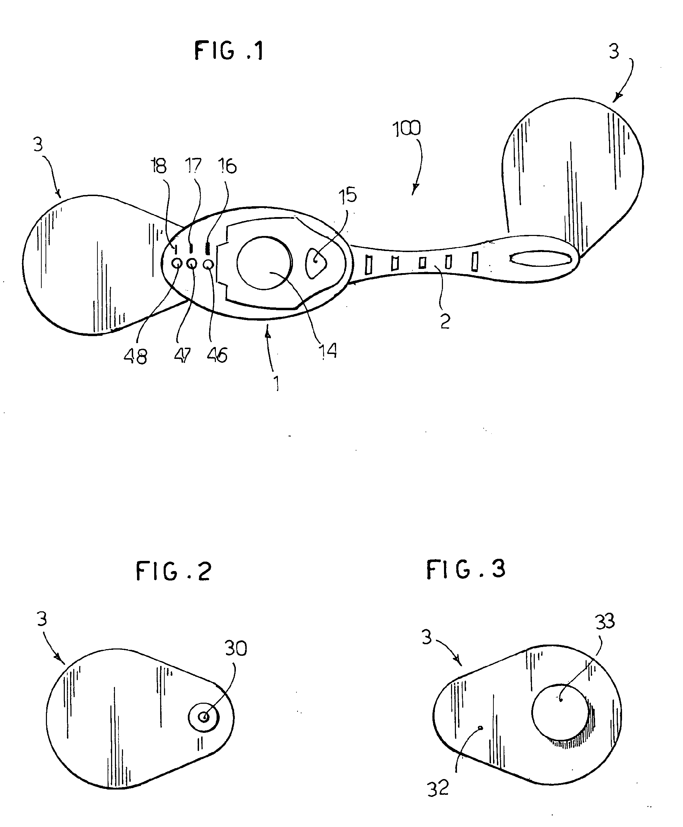

Figure 1 is a top plan view of the heart rate measuring and storage device according to the invention;
Figure 2 is a top plan view of an electrode illustrating the surface destined to face outwards;
Figure 3 is a bottom plan view of an electrode illustrating the surface destined to face towards the user's skin;
Figure 4 is a sectional view illustrating the heart rate measurement and storage device according to the invention exploded;
Figure 5 is a sectional view of the device of Figure 4 assembled; and
Figure 6 is a block diagram illustrating the electrical circuit of the heart rate measuring and storage device according to the invention.

The heart rate measurement and storage device according to the invention, indicated as a whole with reference numeral 100, is described with the aid of the figures.

The device 100 comprises a central body consisting of a substantially elliptical shaped case 1 of rigid material connected to a substantially rectangular tentacle or tail 2 of semi-rigid flexible material. Two substantially circular electrodes 3 are connected to the case 1 and to the free end of the tentacle 2.

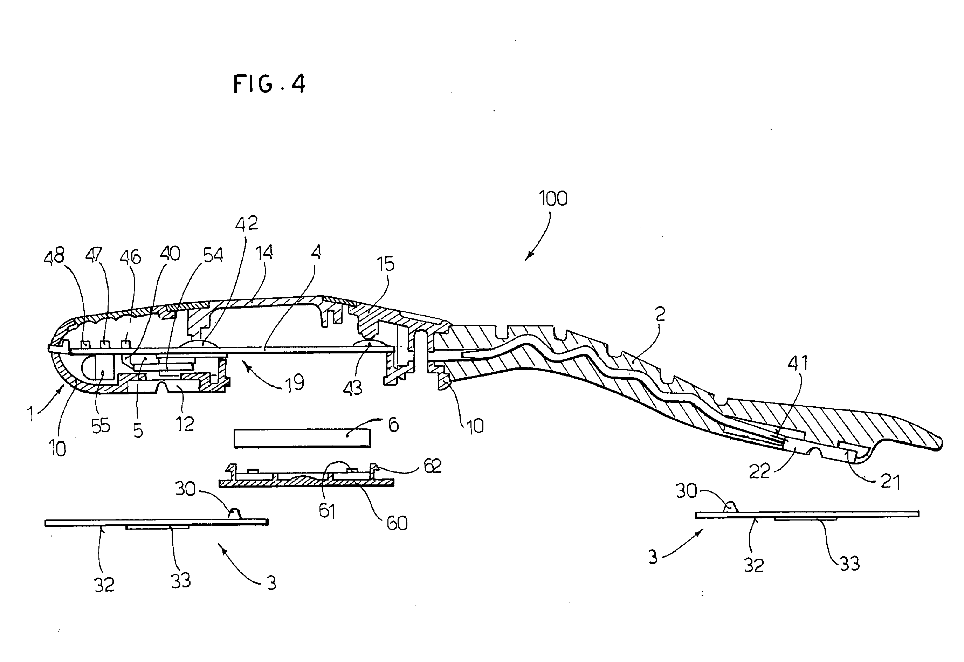

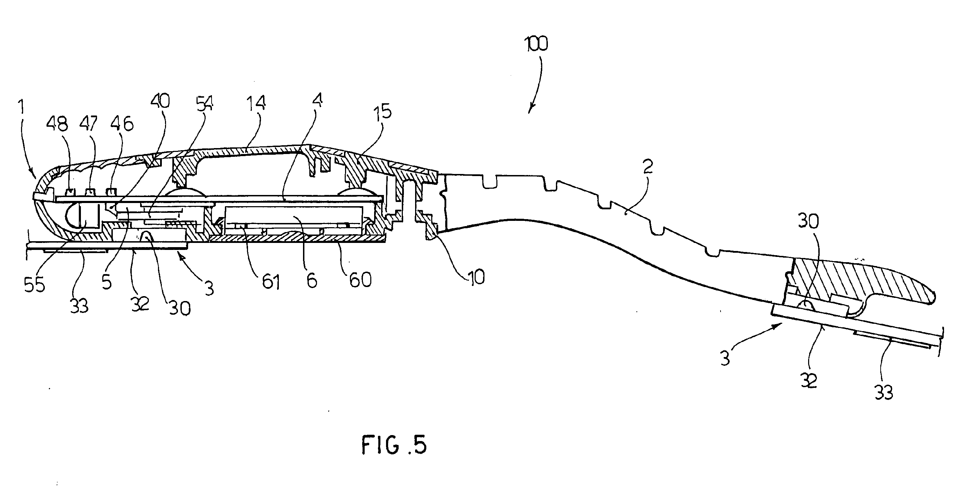

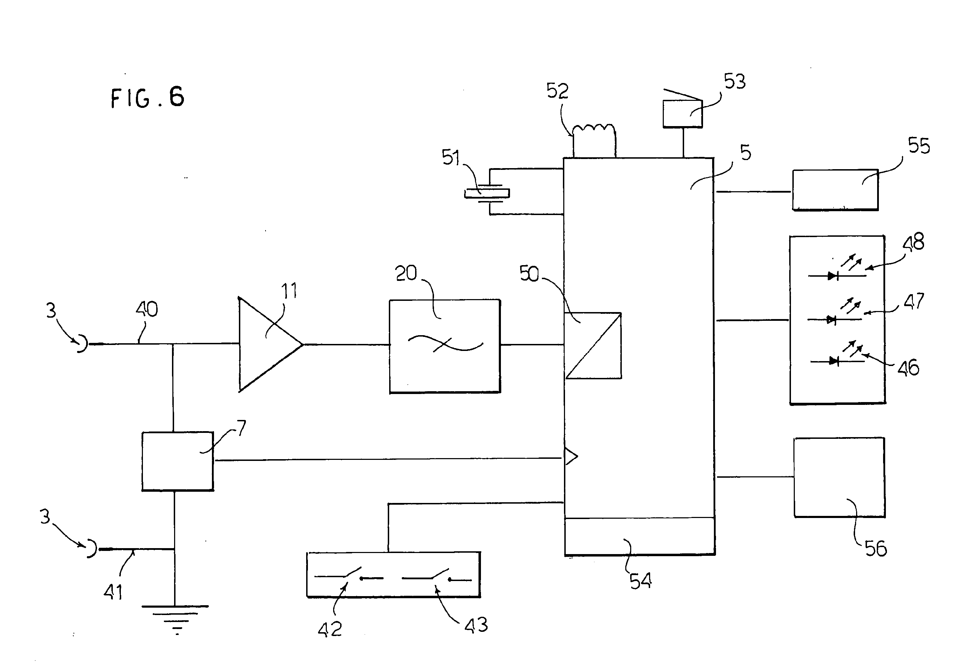

As shown better in Figures 4 and 5, the case 1 comprises a housing of rigid material 10. Contained within the housing 10 is a printed circuit board or card 4 on which are mounted a microcontroller or integrated circuit 5 which manages all functions of the device 100 and a memory 56 (Fig. 6) in which the data detected by the device 100 and set by the user can be stored.

Hereinafter the terms bottom and top will indicate respectively the surface of the device destined to face towards the patient's skin and the surface of the device destined to face outwards.

A retaining spring clip 12 which defines a downwardly open recessed seat is fixed in the bottom wall of the case 1.

A seat 21 in which is fixed a retaining spring clip 22 which defines a downwardly open recessed seat is formed in the bottom wall of the end of the tentacle 2.

The top surface of each electrode 3 has a snap fastener stud 30 which protrudes upwards to be inserted in the special seats of the clips 12 and 22 situated on the bottom surface of the case 1 and at the end of the tentacle 2. In this manner the studs 30 of the electrodes 3 couple, in a snap coupling relationship, within the seats of the spring clips 12 and 22.

The studs 30 of the electrodes and the spring clip 12 and 22 are made of electrically conductive material, since they must act as electrical contacts for transmission of the electrical signal detected by the electrodes 3 towards the microcontroller 5 of the circuit card 4. In fact, in order to acquire the electrical signal generated by the difference in potential between the two electrodes 3, a first electrical cable 40 disposed inside the housing 10 connects the first spring 12 to the circuit card 4 and a second electrical cable 41, passing through the tentacle 2, connects the second spring 22 to the circuit card 4.

The electrodes 3 are disposable and of the stress test ECG type normally available commercially. The bottom surface of the electrode 3 has an adhesive material 32 able to adhere to the user's skin. Therefore before use the bottom surface of the electrode is covered with a removeable sheet of plastic. This sheet must be removed in order to allow the electrode to adhere to the person's skin.

Again on the bottom surface of each electrode 3, a round central area filled with a conductive gel 33 which is in contact with the button 30 is provided. The centre of the conductive area 33 is preferably not coaxial with the snap fastener stud 30. This allows rotation of the electrode 3 around the axis of the snap fastener stud 30 in order best to position the conductive area 33 and to obtain the best electrical signal. The adhesive surface 32 is not conductive and is disposed around the conductive gel 33.

Two push buttons 14 and 15 are inserted in the top wall of the case 1. The push buttons 14 and 15 operate respective switches 42 and 43 mounted on the circuit card 4 and can be actuated even when the device 100 is worn under winter clothes.

The first push button 14 is devoted to turning the device 100 on and off, to record event markers and, in combination with the second push button 15, allows the device 100 to be set. The second push button 15 is used to activate the set functions of the device 100.

Three LED 46, 47 e 48 are mounted on the circuit card 4 and are visible through respective transparent windows, formed on the top wall of the case 1 in register with the LED 46, 47 and 48. The LED 46, 47 and 48 provide the user with information about the heart rate measurement and storage modalities. Three heart rate measurement modalities are provided:
1) Beat to beat,
2) Average of times calculated between four consecutive beats, and
3) Average of times calculated between eight consecutive beats.
   According to which of the three LEDs 46, 47 e 48 flashes, the user can know with which working modality the device 100 has been configured.

Moreover on the top wall of the case 1, level with the windows of the LEDs, a set of three notches 16, 17 and 18 of different thicknesses and decreasing height are engraved and provide the user with information about the residual memory capacity in the memories 56 available for data recording.

In the bottom part of the case 1 an aperture 19 is provide into which an electrical power battery 6 can be inserted, such as a lithium batter, which serves to power all the electronic components of the device 100. The aperture 19 of the case 1 is closed by means of a cover 60 provided with a compression element 61 for the battery 6. An O-ring 62 is disposed around the neck of the cover 60 to ensure a tight seal of the inside of the housing 10. In this manner the device 100 can also be worn by swimmers in fresh water, provided the depth of the water does not exceed 5 meters.

With reference to Figure 6 the electrical diagram of the device 100 is illustrated. An electrical signal sensor 7 which receives the electrical signals detected by the two electrodes 3 disposed on the user's skin and transmits the difference in potential between the two electrodes 3 to the microcontroller 5 is mounted on the circuit card 4.

A signal amplifier 11 and an analogical filter 20 are connected to one of the two electrodes 3. Thus the signal coming from said electrodes 3 is amplified, filtered and sent to an analog-to-digital converter 50 which transforms it into a digital signal for processing by the microcontroller 5. Thus all the information relating to the heart rate is stored on the memory 56, which is preferably a flash memory.

A crystal 51 which acts as a clock for the timing functions of the microcontroller is connected to the microcontroller 5.

A piezoelectric buzzer 53 disposed inside a 2-mm cavity beneath the push button 14, which acts as a sound box, is connected to the microcontroller 5. The buzzer 53 acts as an alarm device with acoustic signalling.

A temperature sensor which detects the temperature of a conductive paste or metal foil 54, interposed between the microcontroller 5 and the clip 12 for coupling the electrode 3, is incorporated into the microcontroller 5. In this manner, the temperature sensor is able to detect the temperature around the electrode 3 applied to the user's body surface. The temperature value is stored in the flash memory 56.

An infrared receiver-transmitter 55 is connected to the microcontroller 5 to download data stored on a PC, provided with an infrared port, and transfer the content of the memory 56 into an acquisition device, such as a PC with an infrared port. As shown in Figures 4 and 5, this infrared transmitter 55 can be disposed in the front part of the case 1, in register with a transparent window formed at the end of the head of the case 1.

Lastly, a wireless transmitter 52, provided with an emitter inductance able to transmit a wireless signal, indicative of the heart rate, at a frequency of about 5 KHz, to a wireless receiver, such as for example the receiver of known devices such as wrist watches, to process and display the heart rate, can be connected to the microcontroller 5.

From this detailed description the advantages of the device 100 according to the invention are evident.

The device 100 does not need a ring-like strap to adhere to the person's torso. This is achieved thanks to the pair of disposable electrodes 3 normally available commercially. Once a protective layer of adhesive plastic on the bottom surface of each electrode 3 has been removed, the electrodes 3 can be made to adhere to the person's chest in the optimal position, independently of the shape of the individual's chest.

The device 100 does not oblige the user to wear a second device (typically a wrist watch) which works as a data memory and as a man-machine interface. In fact many useful functions are implemented inside the electronic circuit 5 of said device 100, which can be configured in order to receive and execute commands. Nevertheless, a standard commercial watch able to receive a wireless signal at 5 KHz can in any case be used in combination with the described device 100.

The commands which can be received and executed by the device 100 are related to:
1) the heart rate measuring and storing modality (beat to beat, that is to say the interval of time that elapses between one beat and the next, the average of the intervals of time calculated over four consecutive beats, the average of the intervals of time calculated over eight consecutive beats).
2) Warnings that the pre-set upper and lower heart rate thresholds have been exceeded. These warnings are given by an acoustic alarm generated by the piezoelectric buzzer 53 provided with a sound box.
3) Recording of event markers (at the user's command, events are stored which can be significant for the subsequent heart rate analyses, such as for example the beginning of a lap, the change in rhythm of the exercise, etc.).

Thus the user can record in the memory 56 the heart rate values and other significant parameters (such as the body temperature detected by the sensor 54, the pre-set alarm thresholds, or markers of particular events that have occurred during the exercise). The user can record the course of these parameters throughout the physical exercise and for a number of sessions, until the memory 56 of the device is full.

Numerous changes and modifications of detail within the reach of a person skilled in the art can be made to the present embodiment of the invention without departing from the scope of the invention, as set forth in the appended claims.

## Claims

1. A device (100) for measuring and storing a user's heart rate for sports activities, physical exercise and rehabilitation, **characterized in that** it comprises:
- a rigid case (1) containing a printed circuit card (5) on which are mounted a microcontroller (5) and a memory (56);
- a tentacle (2) of semi-rigid flexible material, connected to said rigid case (1);
- two electrodes (3) for stress test electrocardiograms having an adhesive bottom surface (32) to adhere to the user's skin and connecting means (30) disposed on the top surface to connect removeably with complementary connecting means (12, 22) disposed respectively in the rigid case (1) and at the end of the tentacle (2),
- said electrodes (3) having an electrically conductive area (33) connected electrically to said microcontroller (5) to measure the electrocardiographic signal (ECG) by means of the difference in potential that is generated between the two electrodes (3).

2. A device (100) according to claim 1, **characterized in that** it comprises an electrical signal sensor (7) connected to the electrodes (3) and to the microcontroller (5) to detect the heart beat on the basis of the electrical signal present on the user's skin, which is detected by the electrodes (3).

3. A device (100) according to claim 1 or 2, **characterized in that** each of said electrodes comprises an area filled with conductive gel (33) around which an adhesive and non conductive bottom surface (32) is provided.

4. A device (100) according to any one of the preceding claims, **characterised in that** said electrode connecting means (3) comprise an electrical connector element (30) connected to the conductive part (33) of the electrode which engages removeably with a respective complementary connecting element (12, 22) fixed in the case (1) and at the end of the tentacle (2) and connected by means of electrical cables to said microcontroller (5).

5. A device (100) according to claim 4, **characterized in that** said connecting element of the electrode (3) is a snap fastener stud (30) which engages, in a snap coupling relationship, with a respective clip spring (12, 22) provided in the case (1) and at the end of the tentacle (2).

6. A device (100) according to any one of the preceding claims, **characterized in that** mounted in said case (1) are an on/off pushbutton (14) and a setting pushbutton (15) connected to respective switches (42, 43) mounted on the circuit card (4) to control said microcontroller (5) so that the user can configure the operating modalities of said device in order to store the heart rate in the memory (56) with three different levels of accuracy (beat to beat, average over four beats, average over eight beats).

7. A device (100) according to any one of the preceding claims, **characterized in that** mounted on said circuit card (4) are three LEDs (46, 47 48) controlled by said microcontroller (5) and disposed in register with three respective transparent windows formed in the case (1), so that the user can verify the operating modalities selected through the luminous signal of one of said LEDs, wherein each of said LEDs, when it flashes, provides two different items of information: the presence of the heart beat signal; the operating modality configured out of three different possible modalities (beat to beat, average over four beats, average over eight beats).

8. A device (100) according to claim 7, **characterized in that** notches (16, 17, 18) of decreasing thickness and height disposed in register with the windows of said LEDs (46, 47, 48) are made on the case (1) to allow the user to check the residual memory capacity of said memory (56).

9. A device (100) according to any one of the preceding claims, **characterized in that** it comprises a temperature sensor incorporated in the microcontroller (5) able to detect the temperature of a conductive paste or metal foil (54) interposed between the microcontroller (5) and the connecting means (12) of the electrode (3), to have an indication of the temperature around the electrode (3) applied to the user's body skin.

10. A device (100) according to any one of the preceding claims, **characterized in that** it comprises a standard data transfer interface (55) mounted on said card (4) and connected to said microcontroller (5) which allows the data stored in said memory (56) to be downloaded to a PC for subsequent processing, analysis and comparison by means of special software provided with the device, said standard interface (55) being an infrared port (IRDA).

11. A device (100) according to any one of the preceding claims, **characterized in that** it comprises a wireless transmitter (52) mounted on said card (4) and connected to said microcontroller (5) interoperable in wireless mode with standard display devices (typically watches to be worn on the wrist) to show the instant heart rate value.

12. A device (100) according to any one of the preceding claims, **characterized in that** it comprises a piezoelectric buzzer (53) disposed within a cavity, beneath a pushbutton (14), which acts as a sound box, which emits an acoustic alarm signal when the heart rate values detected exceed pre-set threshold values.
